# EUROPEAN PATENT APPLICATION

(11) **EP 1 995 248 A1**
(43) Date of publication of application: **26.11.2008**
(21) Application number: 08156601.0
(22) Date of filing: 20.05.2008
(51) Int. Cl.: C07D 487/04

(54) **Process for the production of amino alcohols by asymmetric hydrogenation**

(30) Priority: 23.05.2007 EP 07108709
(71) Applicant: Evonik Degussa GmbH, 45128 Essen (DE)
(72) Inventor: Almena, Juan, 63454, Hanau (DE); Geiß, Gerhard, 65719, Hofheim (DE); Kadyrov, Renat, 60389, Frankfurt (DE)

(57) **Abstract**

The present invention is directed to a process for the production of enantiomerically enriched amino alcohols. Especially, the process is concerned with a catalytic reduction using chiral ferrocenyl ligands. The amino alcohols are generated from compounds comprising the general structure (I).

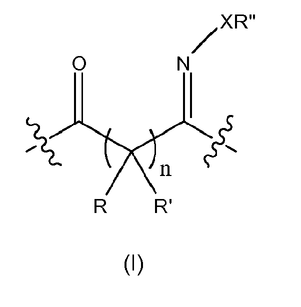

## Description

The present invention is directed to a process for the production of enantiomerically enriched amino alcohols. Especially, the process is concerned with a catalytic reduction using chiral ferrocenyl ligands. The amino alcohols are generated from compounds comprising the general structure (I).

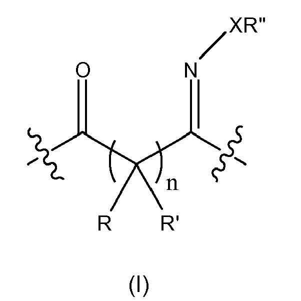

Amino alcohols are versatile tools in organic synthesis. In particular the enantiomerically enriched compounds are of great interest in chemical industry due to being precious starting materials, e.g. for chiral ligands or bioactive compounds. Therefore, there have been many attempts in the scientific community dealing with the production of deemed compounds, either through racemic resolution methods or through stereoselective catalytic processes.

For example in patent JP2001106664 the use of homogeneous catalysts is described for the hydrogenation of ketoximes in two steps:
- the first step is the reduction of the keto group with a homogeneous catalyst;
- a second step is the reduction of the oxime group with borane agents.

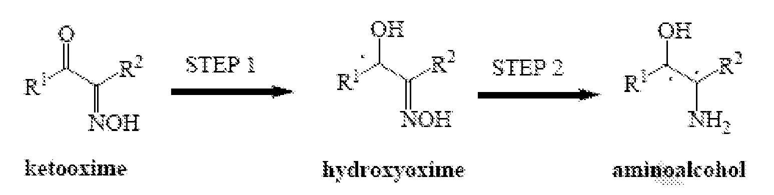

As opposed to a one-step process that uses the same reaction conditions for both reactions, JP2915161 discusses the use of different reaction conditions for each reaction. It characterizes the first step as a hydrogenation that may be carried out with hydrogen in the presence of a monovalent rhodium complex and a ligand. The ligand corresponds in structure to the following formula:

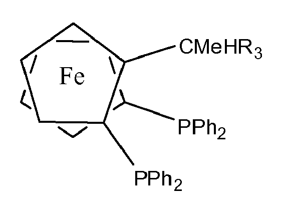

JP2915161 characterizes the second step as a reduction that may be carried out using a metal hydride or using hydrogenation with H₂ in the presence of a Raney nickel or rhodium catalyst.

Both procedures seem inappropriate from a technological and economical point of view. It is mandatory to perform the reaction in two steps for using incompatible reducing agents in each of the steps. On the other hand the use of the borane reducing agent in equimolar amounts is disadvantageous on a technical scale production.

The use of chiral metal complexes containing enantiomerically enriched ferrocenyl ligands has long been known (Togni, A.; Hayashi, T. Editors. Germany (1995), 105-142 pp. Publisher: (Wiley-VCH Verlag GmbH & Co. KGaA, Weinheim, Germany). Especially, in US7,015,342, US6284925, EP114095, US6,348,620 and US7,009,065 the applicability of ferrocenyl ligands in enantioselective hydrogenation is demonstrated.

The object of the present invention was, therefore, to find another way to produce enantioselective amino alcohols. In particular, this method should serve for a superior production of these compounds on a technical scale under the perspective of ecological and economical aspects starting from inexpensive easily accessible precursors.

The object is met by performing a process depicted in claim 1. Other preferred embodiments of the process according to the invention are protected in the following subclaims 2 to 11.

The set object is, completely surprisingly and extremely advantageously, achieved by applying a process for the enantioselective production of amino alcohols, wherein compounds comprising the general structure (I) 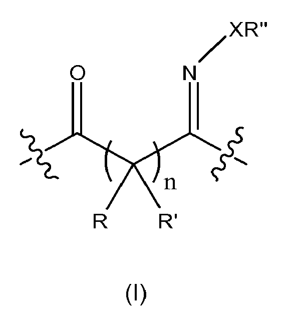 wherein
n is an integer from 0 to 4
X is O or NH
R, R', R" is selected from the group of
H, (C₁-C₁₅)-alkyl, (C₂-C₁₅)-alkenyl, (C₂-C₁₅)-alkynyl, (C₂-C₁₅)-alkoxyalkyl, (C₆-C₁₈)-aryl, (C₇-C₁₉)-aralkyl, (C₃-C₁₈)-heteroaryl, (C₄-C₁₉)-heteroalkyl, (C₁-C₈)-alkyl-(C₆-C₁₈)-aryl, (C₁-C₈)-alkyl-(C₃-C₁₉)-heteroaryl, (C₃-C₈)-cycloalkyl, (C₁-C₈)-alkyl-(C₃-C₈)-cycloalkyl or (C₃-C₈)-cycloalkyl-(C₁-C₈)-alkyl, (C₁-C₈)-Acyloxy, (C₁-C₈)-Acyl,
are reduced in the presence of hydrogen and a metal catalyst comprising a catalytically active metal selected from the group consisting of Ru, Rh, Ir, Pd, Pt and an enantiomerically enriched bidentate ferrocenyl ligand provided that the amino alcohol is not 6-amino-7-hydroxy-4,5,6,7-tetrahydro-imidazo[4,5,1-3K][1]-benzazepin-2[1H]-on or salts thereof, the skilled worker is able to generate the desired compounds in an easy to handle and a particular favourable manner. The teaching of the present invention enables the artisan to both obtain the amino alcohol with high enantiomeric excess and in a one pot/step reaction. This was neither anticipated nor taught to be possible through the prior art. In this respect, it is not allowed to assume that the reaction according to the invention is obvious.

The present invention is preferably performed using compounds of the general formula (I) having at least one of the following features selected from the group:
n is 0 or 1,
X is O,
R" is H,
R, R' are selected from being H, (C₁-C₁₅)-alkyl, (C₆-C₁₈)-aryl.

It is a further preferable aspect of the present invention to use ferrocenyl ligands of general formula (II) 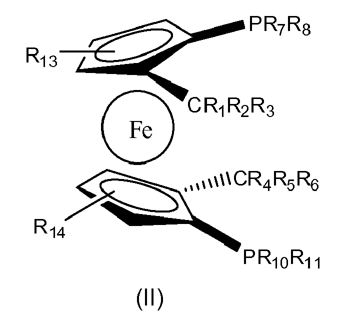 wherein
R₁, R₂, R₃, R₄, R₅, R₆ are selected independently of each other from the group of H, (C₁-C₁₅)-alkyl, (C₂-C₁₅)-alkenyl, (C₂-C₁₅)-alkynyl, (C₂-C₁₅)-alkoxyalkyl, (C₆-C₁₈)-aryl, (C₇-C₁₉)-aralkyl, (C₃-C₁₈)-heteroaryl, (C₄-C₁₉)-heteroalkyl, (C₁-C₈)-alkyl-(C₆-C₁₈)-aryl, (C₁-C₈)-alkyl-(C₃-C₁₉)-heteroaryl, (C₃-C₈)-cycloalkyl, (C₁-C₈)-alkyl-(C₃-C₈)-cycloalkyl or (C₃-C₈)-cycloalkyl-(C₁-C₈)-alkyl, OR, NRR',
R₇, R₈, R₁₀, R₁₁, are selected independently of each other from the group of isopropyl, tert.-butyl, neopentyl, substituted or unsubstituted (C₆-C₁₈)-aryl, substituted or unsubstituted (C₇-C₁₉)-aralkyl, substituted or unsubstituted (C₁-C₈)-alkyl-(C₆-C₁₈)-aryl, substituted or unsubstituted cyclopentyl, substituted or unsubstituted cyclohexyl, substituted or unsubstituted adamantyl.
R₁₃, R₁₄ are selected independently of each other from the group of H, (C₁-C₁₅)-alkyl, (C₂-C₁₅)-alkoxyalkyl, (C₆-C₁₈)-aryl, (C₇-C₁₉)-aralkyl, (C₃-C₁₈)-heteroaryl, (C₄-C₁₉)-heteroalkyl, (C₁-C₈)-alkyl-(C₆-C₁₈)-aryl, (C₁-C₈)-alkyl-(C₃-C₁₉)-heteroaryl, (C₃-C₈)-cydoalkyl, (C₁-C₈)-alkyl-(C₃-C₈)-cycloalkyl or (C₃-C₈)-cycloalkyl-(C₁-C₈)-alkyl, (C₁-C₈)-Acyloxy, (C₁-C₈)-Acyl,
or display a (C₃-C₅)-alkylene bridge or an annelated aromatic or aliphatic ring system.

In a preferable embodiment the invention is performed using an enantiomerically enriched ligand of the following structure: 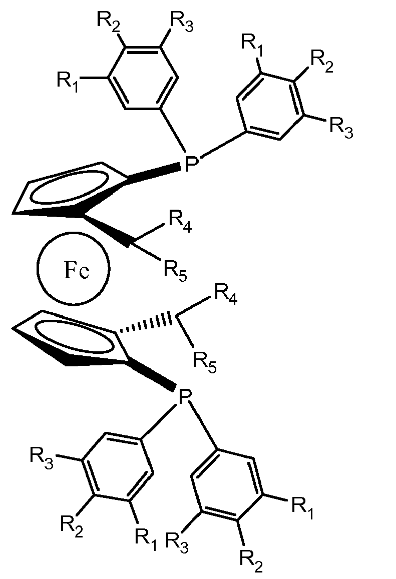 wherein R₁, R₂, R₃ independently can be H, (C₁-C₈)alkyl, (C₁-C₈)alkoxy, R₄ and R₅ are independently chosen from the group NR₆R₇, substituted or unsubstituted (C₆-C₁₈)-aryl, substituted or unsubstituted (C₄-C₁₉)-heteroalkyl, (C₁-C₁₅)-alkyl; R₆ and R₇ can independently of each other be H, (C₁-C₁₅)alkyl, substituted or unsubstituted (C₆-C₁₈)-aryl, substituted or unsubstituted (C₄-C₁₉)-heteroalkyl.

In a more preferred manner the process according to the invention is performed using the following enantiomerically enriched ligand:

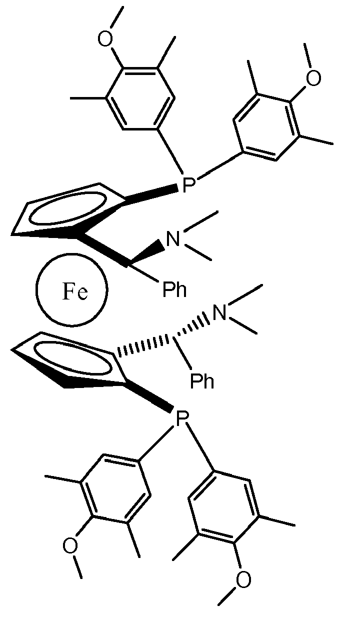

The preparation of the metal-ligand complexes just shown by way of example can be effected in situ by reaction of a metal salt or of a corresponding precomplex with the ligands of the general formula (II). In addition, a metal-ligand complex can be obtained by reacting a metal salt or an appropriate precomplex with the ligands of the general formula (II) and subsequent isolation.

Preferably the metal used for building up the catalyst is selected from the group of rhodium, ruthenium and iridium.

Examples of metal salts to build up the active catalyst are metal chlorides, bromides, iodides, cyanides, nitrates, acetates, acetylacetonates, hexafluoroacetylacetonates, tetrafluoroborates, hexafluorophosphates, hexafluoroantimonates, perfluoroacetates or triflates, especially of rhodium, ruthenium, iridium.

Examples of the precomplexes to build up the active catalyst are, for example: Cyclooctadienepalladium chloride, cyclooctadienepalladium iodide, palladium(II) acetate, palladium(II) bromide, palladium(II) chloride, 1,5-hexadienepalladium chloride, 1,5-hexadienepalladium iodide, bis(dibenzylideneacetone)palladium, bis(acetonitrile)palladium(II) chloride, bis(acetonitrile)palladium(II) bromide, bis(benzonitrile)palladium(II) chloride, bis(benzonitrile)palladium(II) bromide, bis(benzonitrile)palladium(II) iodide, bis(allyl)palladium, bis(methallyl)palladium, allylpalladium chloride dimer, methallylpalladium chloride dimer, tetramethylethylenediaminepalladium dichloride, tetramethylethylenediaminepalladium dibromide, tetramethylethylenediaminepalladium diiodide, tetramethylethylenediaminepalladium dimethyl,
cyclooctadieneplatinum chloride, cyclooctadieneplatinum iodide, 1,5-hexadieneplatinum chloride, 1,5-hexadieneplatinum iodide, bis(cyclooctadiene)platinum, potassium ethylenetrichloroplatinate,
cyclooctadienerhodium(I) chloride dimer, norbornadienerhodium(I) chloride dimer, 1,5-hexadienerhodium(I) chloride dimer, tris(triphenylphosphine)rhodium(I) chloride,
hydridocarbonyltris(triphenylphosphine)rhodium(I) chloride,
bis(norbornadiene)rhodium(I) perchlorate, bis(norbornadiene)rhodium(I) tetrafluoroborate, bis(norbornadiene)rhodium(I) triflate,
bis(acetonitrilecyclooctadiene)rhodium(I) perchlorate, bis(acetonitrilecyclooctadiene)rhodium(I) tetrafluoroborate, bis(acetonitrilecyclooctadiene)rhodium(I) triflate,
bis(acetonitrilecyclooctadiene)rhodium(I) perchlorate, bis(acetonitrilecyclooctadiene)rhodium(I) tetrafluoroborate, bis(acetonitrilecyclooctadiene)rhodium(I) triflate, 1,5-cyclooctadienerhodium(I) acetoacetonate salts with halide, triflate, tetrafluoroborate, perchlorate anions,
cyclopentadienerhodium(III) chloride dimer, pentamethylcyclopentadienerhodium(III) chloride dimer,
(cyclooctadiene)Ru(η³-allyl)₂, ((cyclooctadiene)Ru)₂(acetate)₄, ((cyclooctadiene)Ru)₂(trifluoroacetate)₄, RuCl₂(arene) dimer, (Ruarenel₂)₂, tris(triphenylphosphine)ruthenium(II) chloride, cyclooctadieneruthenium(II) chloride,
cyclooctadieneiridium(I) chloride dimer, bis(cyclooctene)iridium(I) tetrafluoroborate.

The complexes based on one or more metals of the metallic elements and ligands of the general formula (II), especially from the group of Ru, Rh, Ir, Pd, Pt may already be catalysts or be used to prepare the catalysts based on one or more metals of the metallic elements, especially from mentioned group. For examples the catalysts may - prior to its use - be transformed to the active form via reduction or oxidation processes known to the artisan. Preferably the metal used is selected from the group of rhodium, ruthenium and iridium. Mostly preferred is Rh. More preferably the metal catalyst is obtained from bis(cyclooctadiene)rhodium(I) tetrafluoroborate, bis(cyclooctadiene)rhodium(I) hexafluorophosphate, bis(cyclooctadiene)rhodium(I) hexafluoroantimonate or bis(norbornadiene)rhodium(I) tetrafluoroborate.

With respect to the amount of catalyst to be employed based on the substrate, it can be noted that in principle a higher amount of catalyst also helps to achieve better conversions. On the other hand the catalyst proves to be a costly component. Therefore, the artisan has to set the amount of catalyst to be applied in terms of an economic optimum. The amount of catalyst which has to be employed is preferably in the range from 0.001 mol% to 10 mol% based on the substrate employed. Further preferably, the use of the catalyst lies in the range from 0.01 mol% to 1 mol% and very particularly preferably in the range from 0.05 mol% to 0.5 mol%

The temperature which is to be set during the reaction can be determined by the person skilled in the art. It should be so high that the envisaged reaction proceeds in sufficiently quick time, but if possible so low that the by-product spectrum can be kept as low as possible in the reaction according to the invention. It has turned out that a temperature of greater than 20°C and less than 100°C is to be preferred. A temperature range of greater than 30°C and less than 60°C is very particularly preferred. Utmost preferred is a temperature between app. 30°C and 50°C.

The pressure of hydrogen applied during the present invention may be fixed by the skilled worker according to needs already discussed for the temperature. Preferably hydrogenation is effected at hydrogen pressure of 0.1 to 10 MPa, preferably 0.2 to 8 MPa and most preferably at 2 - 6 MPa.

In principle, the person skilled in the art is free in the choice of the solvent in which he would like to carry out the reaction according to the invention. On account of the fact that the starting substances are often present in liquid form, in this respect the use of a solvent can also be dispensed with. If, however, the use of solvents in the reaction according to the invention is desired, it is advantageous to use those solvents which dissolve the employed components of the reaction correspondingly well and otherwise prove inert to the reaction according to the invention. Those suitable are hydrocarbons, in particular aromatic hydrocarbons and their perfluorinated derivatives, ethers, carboxylic acid amides, carboxylic acid esters, thioethers, carbonates, nitriles, halogenated benzenes, and the like. Other inert solvents are ionic or supercritical liquids, which should already be known to the person skilled in the art (for ionic liquids: Wasserscheid, P.; Welton, T.; Editors. Germany. (2003), 364 pp. Publisher: (Wiley-VCH Verlag GmbH & Co. KGaA, Weinheim, Germany); for supercritical liquids: Jessop, P.G.; Ikariya, T. Noyori, R. Science 1995, 269, 1065-1069; Chem Rev 1999, 99, 475-493; Chem. Commun. 1999, 1235-1236).
Preferred solvents are those selected from the group consisting of hexane, heptane, octane, NMP (N-methylpyrrolidone), DMGE (dimethyl glycol ether), alcohols, toluene, anisole, benzene, chlorobenzene, DMF (dimethylformamide), DMAC (dimethylacetamide), 1,4-dioxane, MTBE, THF, acetonitrile, benzonitrile, ethyl acetate, isopropyl acetate, dibutyl ether, dimethyl carbonate, diethyl carbonate, dipropyl carbonate and the like.
Extremely preferred are solvent like methanol, ethanol, isopropanol, toluene, dimethylacetamide, THF, dichloromethane, dimethylformamide or mixtures thereof.

In another aspect the present invention is directed to a process according to the invention, wherein an acidic or basic additive is present during the reduction.
Preferably, this, however, can be inexpensive inorganic or organic bases or acids.
Suitable inorganic bases preferably to be employed are, in particular, carbonates, hydrogencarbonates, phosphates, mono- or dihydrogenphosphates or hydroxides of the alkali metals or alkaline earth metals. Very particularly preferred are those selected from the group consisting of K₃PO₄, K₂HPO₄, K₂CO₃, Cs₂CO₃, NaOH, KOH, and the like.
Organic bases preferably to be employed are alkali metal or alkaline earth metal salts of weak organic acids, such as, for example, acetic acid, formic acid, propionic acid, and the like. Further organic bases preferably to be employed are nitrogen-containing organic molecules such as, for example, those selected from the group consisting of NEt₃, N(n-Bu)₃, DABCO® (1,4-diazabicyclo[2,2,2]octane), DBU® (1,8-diazabicyclo[5,4,0]undec-7-ene, N,N-dimethylglycine ethyl ester, pyridine, tetramethylguanidine TMEDA, hexamethylenetetramine, and the like. Further preferred bases are amines supported on oligomers and polymers and their derivatives (such as, for example, guanidines).
Suitable inorganic acids are acids which possess an anion which coordinates only weakly, like for example sulphuric acid, tetrafluoroboric acid, hexafluorophosphoric acid, hydrogenbromide, hydrogen iodide.
Suitable organic acids are for example, acetic acid, trifluoroacetic acid, formic acid, propionic acid, and the like.

In the enantioselective hydrogenation, a procedure is preferable which aims to dissolve substrates to be hydrogenated and complex/catalyst in a solvent. As indicated above, the catalyst is preferably formed from a precatalyst in the presence of the chiral ligand by reaction or by prehydrogenation before the substrate is added. Subsequently, hydrogenation is affected at the appropriate pressure and within the temperature limits given.
The ratio of substrate to catalyst is determined by economic considerations. The reaction should proceed sufficiently rapidly with minimum complex/catalyst concentration. However, preference is given to working at a substrate/catalyst ratio between 50 000:1 and 10:1, preferably 1000:1 and 50:1.
The reaction is preferably performed as a one-pot-process, optionally with one catalyst.

At the time of the invention, it was by no means obvious that the process presented here permit the easy generation of enantiomerically enriched amino alcohols under present conditions compared to the known prior art systems and simultaneously allow to outperform the advantageous properties and capabilities of the prior art systems. The inventive process features high chiral induction in the underlying catalysis in an easy to handle and safe one-step/one-pot process, especially with only one catalyst, and without the need to isolate e.g. any partially reduced intermediates.

(C₁-C₈)-Alkyl radicals are to be regarded as methyl, ethyl, *n*-propyl, isopropyl, *n*-butyl, isobutyl, *sec*-butyl, *tert*-butyl, pentyl, hexyl, heptyl or octyl together with all of their bonding isomers.
A (C₁-C₁₅)-alkyl radical in the context of the definition according to the invention is an appropriate radical having 1 to at most 15 C atoms.
The radical (C₁-C₈)-alkoxy corresponds to the radical (C₁-C₈)-alkyl with the proviso that this is bonded to the molecule via an oxygen atom.
(C₂-C₈)-Alkoxyalkyl means radicals in which the alkyl chain is interrupted by at least one oxygen function, it not being possible for two oxygen atoms to be bonded to one another. The number of carbon atoms indicates the total number of carbon atoms contained in the radical.
A (C₃-C₅)-alkylene bridge is a carbon chain having three to five C atoms, this chain being bonded to the molecule considered via two different C atoms.
The radicals just described in the preceding paragraphs can be mono- or polysubstituted by halogens and/or heteroatom-containing radicals having N, O, P, S, Si atoms. These are, in particular, alkyl radicals of the abovementioned type, which have one or more of these heteroatoms in their chain or bonded to the molecule via one of these heteroatoms.
The primary, optionally linear (C₃-C₂₀)-alkyl radical is to be seen in the context of the definition according to the invention as corresponding to the (C₁-C₈)-alkyl radical, but where a branching exists at the earliest on the C₂, more preferably C₃, further preferably C₄, very preferably C₅, atom or radical.

(C₁-C₈)-Acyloxy in the context of the invention is a (C₁-C₈)-alkyl radical as defined above having at most 8 C atoms, which is bonded to the molecule via a COO function.

(C₁-C₈)-Acyl in the context of the invention is a (C₁-C₈)-alkyl radical as defined above having at most 8 C atoms, which is bonded to the molecule via a CO function.

A (C₆-C₁₈)-aryl radical is understood as meaning an aromatic radical having 6 to 18 C atoms. In particular, these include compounds such as phenyl, naphthyl, anthryl, phenanthryl, biphenyl radicals or systems of the type previously described fused to the molecule concerned, such as, for example, indenyl systems, which can optionally be substituted by (C₁-C₈)-alkyl, (C₁-C₈)-alkoxy, (C₂-C₈)-alkoxyalkyl, NH(C₁-C₈)-alkyl, N((C₁-C₈)-alkyl)₂, OH, O(C₁-C₈)-alkyl, NO₂, NH(C₁-C₈)-acyl, N((C₁-C₈)-acyl)₂, F, Cl, CF₃, (C₁-C₈)-acyl, (C₁-C₈)-acyloxy, (C₇-C₁₉)-aralkyl radical, (C₄-C₁₉)-heteroaralkyl.

A (C₇-C₁₉)-aralkyl radical is a (C₆-C₁₈)-aryl radical bonded to the molecule via a (C₁-C₈)-alkyl radical.

A (C₃-C₁₈)-heteroaryl radical in the context of the invention is a five-, six- or seven-membered aromatic ring system of 3 to 18 C atoms, which contains heteroatoms such as, for example, nitrogen, oxygen or sulphur in the ring. Such heteroatoms are in particular to be regarded as radicals such as 1-, 2-, 3-furyl, 1-, 2-, 3-pyrrolyl, 1-, 2-, 3-thienyl, 2-, 3-, 4-pyridyl, 2-, 3-, 4-, 5-, 6-, 7-indolyl, 3-, 4-, 5-pyrazolyl, 2-, 4-, 5-imidazolyl, acridinyl, quinolinyl, phenanthridinyl, 2-, 4-, 5-, 6-pyrimidinyl. The heteroaromatics can be substituted in an identical manner to the abovementioned (C₆-C₁₈)-aryl radicals.

A (C₄-C₁₉)-heteroaralkyl is understood as meaning a heteroaromatic system corresponding to the (C₇-C₁₉)- aralkyl radical.

Halogens (Hal) are fluorine, chlorine, bromine, iodine. Hal' is chlorine, bromine, iodine.

The described chemical structures relate to all the possible stereoisomers which can be attained by altering the configuration of the individual chiral centres, axes or planes that is to all the possible diastereomers and to all the optical isomers, or their mixtures, which form a part thereof.

Within the context of the invention, the term enantiomerically enriched is understood as meaning the proportion, in a range of > 50% and < 100%, of an enantiomer when present in a mixture together with its optical antipode.

Within the context of the invention, the term diastereomeric enrichment is understood as meaning the proportion of a diastereomer, in a range of > 50% and < 100%, when present in a mixture together with its other diastereomers.

The present invention is directed to the transformation of compounds embracing a structure depicted in general formula (I). Hence, it is deemed obvious that these compounds have to be of a nature that the reaction of the invention attacks only the structure of formula (I) in such a compound. The structure of formula (I) can be part of a substituted or unsubstituted open chain molecule or may form a part of a substituted or unsubstituted ring system. The residues attached to the structure and being left open in formula (I) can be any, if applicable substituted or unsubstituted and/or branched chain or linear, H, (C₁-C₁₅)-alkyl, (C₂-C₁₅)-alkenyl, (C₂-C₁₅)-alkynyl, (C₂-C₁₅)-alkoxyalkyl, (C₆-C₁₈)-aryl, (C₇-C₁₉)-aralkyl, (C₃-C₁₈)-heteroaryl, (C₄-C₁₉)-heteroalkyl, (C₁-C₈)-alkyl-(C₆-C₁₈)-aryl, (C₁-C₈)-alkyl-(C₃-C₁₉)-heteroaryl, (C₃-C₈)-cycloalkyl, (C₁-C₈)-alkyl-(C₃-C₈)-cycloalkyl or (C₃-C₈)-cycloalkyl-(C₁-C₈)-alkyl.

### Examples:

### List of ligands

| Nr | Structure | Nr | Structure | Nr | Structure |
|---|---|---|---|---|---|
| 22 | 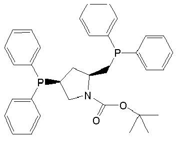 | 591 | 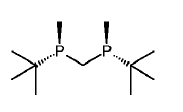 | 688 | 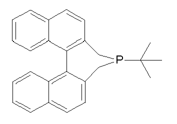 |
| 23 | 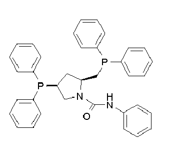 | 597 | 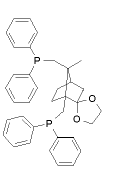 | 731 | 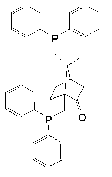 |
| 28 | 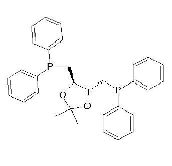 | 601 | 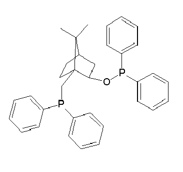 | 732 | 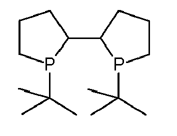 |
| 69 | 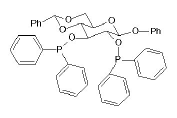 | 607 | 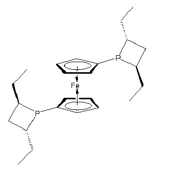 | 826 | 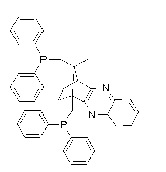 |
| 73 | 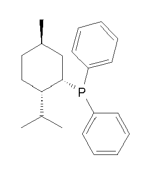 | 614 | 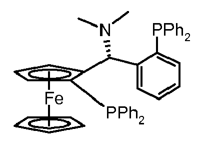 | 939 | 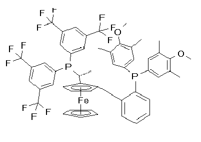 |
| 89 | 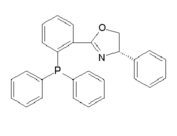 | 615 | 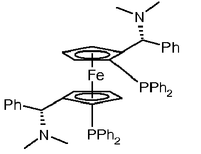 | 979 | 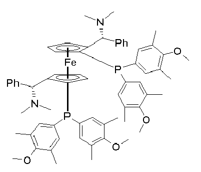 |
| 132 | 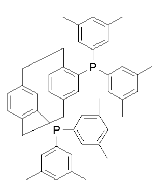 | 621 | 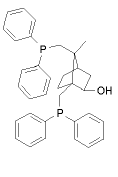 | 1002 | 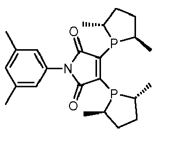 |
| 534 | 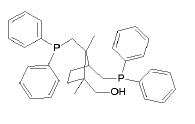 | 627 | 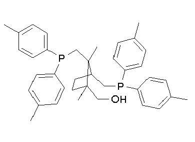 | 1007 | 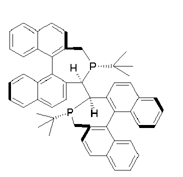 |
| 537 | 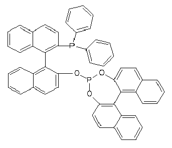 | 664 | 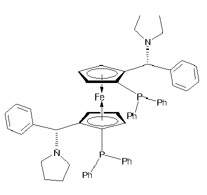 | | |

0.001 mmol of ligand and 0.001 mmol of Rh(COD)₂BF₄ were dissolved under argon in 0.1 ml dichloromethane and the mixture was stirred at room temperature for 10 minutes. In the meantime 0.5 mmol of the ketooxime oximobuzolinone was suspended under argon in 0.5 ml methanol. In case of using an additive like an acid or a base, 0.001 mmol of the corresponding additive was at this point added. The in situ formed catalysts were mixed with the substrate suspension and the reaction mixtures were then introduced into an autoclave and the autoclave was purged with hydrogen. Then 60 bar hydrogen was pressured and the reactions were warmed at 40°C for 20h. After cooling down and release of pressure a sample of the raw mixture (0.1 ml) was taken for analysis [approx. 25 mg Deloxane (metal scavenger) was added to the analytical probe and the suspension was stirred at 50°C for 10 minutes. Then it was filtered through paper and it was diluted with 0.05 ml NaOH 2,5M, 0.5 ml acetonitrile and 0.5 ml water (containing 0,1% formic acid)]. The mixtures were analysed using a HPLC.

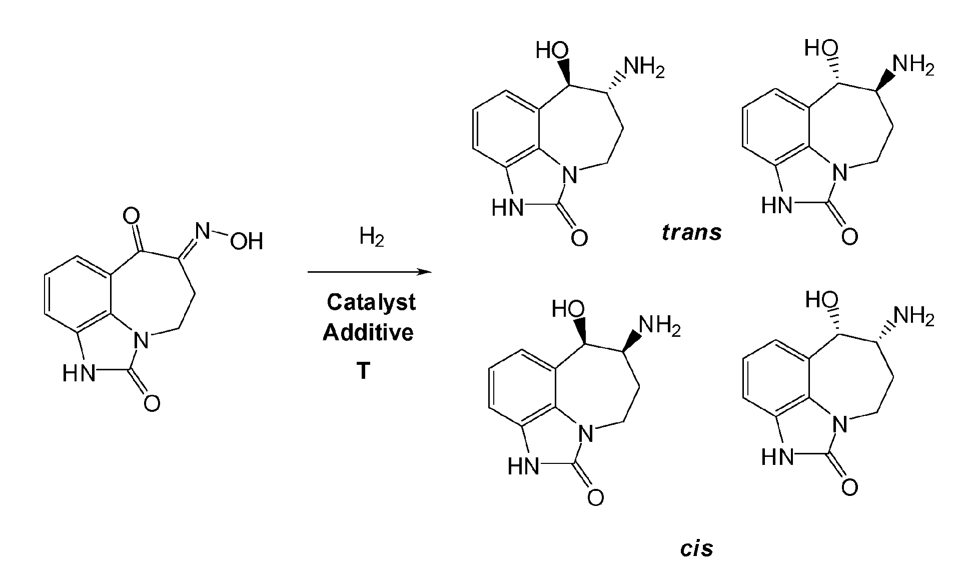

**Table 1: Results with Rh catalysts. The absolute stereochemistry has not been determined. In the table both enantiomers are characterised by positive and negative ee values.**

| **Conv. (%)** | **Aminoalcohol / Hydroxyoxime** | **Trans / cis** | **ee (trans;%)** | **ee (cis;%)** | **Ligand #** | **Solvent** | **Additive** | **P (bar)** | **T (°C)** |
|---|---|---|---|---|---|---|---|---|---|
| 100 | 1/0 | 2,3 | 15 | 4 | **22** | MeOH | KOH | 50 | 40 |
| 100 | 1/0 | 9,9 | 34 | -56 | **28** | MeOH | --- | 50 | 35 |
| 100 | 1/0 | 7,6 | 29 | -63 | **28** | MeOH/THF | --- | 50 | 35 |
| 100 | 1/0 | 4,2 | 29 | -21 | **28** | MeOH | KOH | 50 | 35 |
| 96 | 1/0 | 6,1 | -14 | 76 | **614** | MeOH | --- | 40 | 40 |
| 95 | 1/0 | 127,9 | -78 | -68 | **979** | MeOH | --- | 40 | 40 |
| 92 | 1/0 | 15,9 | -41 | 47 | **23** | MeOH | --- | 50 | 40 |
| 92 | 1/0 | 19,7 | -44 | 51 | **23** | MeOH/ H₂O 10/1 | --- | 50 | 40 |
| 99 | 59,1/1 | 5,6 | 41 | 26 | **28** | MeOH/ H₂O 10/1 | --- | 50 | 40 |
| 100 | 1/0 | 12,1 | 36 | 69 | **28** | MeOH | CF₃CO₂H | 50 | 40 |
| 100 | 1/0 | 10,8 | 35 | 63 | **28** | MeOH/ H₂O 10/1 | CF₃CO₂H | 50 | 40 |
| 100 | 1/0 | 23,8 | 57 | 82 | **597** | MeOH | --- | 50 | 40 |
| 100 | 1/0 | 19,2 | -26 | 53 | **615** | MeOH | --- | 50 | 40 |
| 95 | 1/0 | 29,4 | 52 | 82 | **621** | MeOH | --- | 50 | 40 |
| 99 | 1/0 | 32,2 | 57 | 82 | 597 | MeOH / H₂O 10/1 | --- | 50 | 40 |
| 100 | 1/0 | 18,2 | -26 | 61 | 615 | MeOH / H₂O 10/1 | --- | 50 | 40 |
| 100 | 101,9/1 | 0,8 | -7 | 28 | 627 | MeOH / H₂O 10/1 | --- | 50 | 40 |
| 99 | 1/0 | 25,6 | -32 | 52 | 615 | MeOH | CF₃CO₂H | 50 | 40 |
| 99 | 1/0 | 36,2 | 41 | 82 | **621** | MeOH | CF₃CO₂H | 50 | 40 |
| 100 | 1/0 | 24,2 | -33 | 60 | 615 | MeOH / H₂O 10/1 | CF₃CO₂H | 50 | 40 |
| 99 | 1/0 | 15,7 | 18 | 82 | 621 | MeOH/ H₂O 10/1 | CF₃CO₂H | 50 | 40 |
| 99 | 1/0 | 23,5 | -37 | 35 | 664 | MeOH | --- | 50 | 40 |
| 98 | 1/0 | 30,1 | 29 | 52 | 731 | MeOH | --- | 50 | 40 |
| 96 | 1/0 | 28,5 | 31 | 53 | 826 | MeOH | --- | 50 | 40 |
| 100 | 1/0 | 76,9 | -91 | 8 | 979 | MeOH | --- | 50 | 40 |
| 92 | 1/0 | 40,5 | 31 | 33 | 826 | MeOH/ H₂O 10/1 | --- | 50 | 40 |
| 98 | 1/0 | 58,9 | -44 | 36 | 664 | MeOH | CF₃CO₂H | 50 | 40 |
| 100 | 1/0 | 24,0 | 27 | 0 | 731 | MeOH | CF₃CO₂H | 50 | 40 |
| 99 | 1/0 | 34,6 | 25 | 0 | 826 | MeOH | CF₃CO₂H | 50 | 40 |
| 100 | 1/0 | 24,0 | 71 | 50 | 939 | MeOH | CF₃CO₂H | 50 | 40 |
| 90 | 1/0 | 15,7 | -64 | 0 | 979 | MeOH | CF₃CO₂H | 50 | 40 |
| 93 | 1/0 | 5,3 | -11 | 74 | 731 | iPrOH | CF₃CO₂H | 50 | 40 |
| 99 | 1/0 | 44,8 | 30 | 0 | 939 | AcOEt | CF₃CO₂H | 50 | 40 |

### Reactions done with ruthenium catalysts

0.001 mmol of ligand and 0.0005 mmol of [Ru(C₆H₆)Cl₂]₂ were dissolved under argon in 0.05 ml dimethylformamide and the mixtures were stirred at 100°C for 10 minutes. In the meantime 0.5 mmol of the ketooxime oximobuzolinone was suspended under argon in 0.5 ml of solvent (methanol, THF or mixtures thereof). In case of using an additive like an acid or a base, 0.001 mmol of the corresponding additive was at this point added. The in situ formed catalysts were mixed with the substrate suspension and the reaction mixtures were then introduced into an autoclave and the autoclave was purged with hydrogen. Then the pressure was set as indicated in the table and the reactions were warmed at the temperature given in the table for 20h. After cooling down and release of pressure a sample of the raw mixture (0.1 ml) was taken for analysis [approx. 25 mg Deloxane (metal scavenger) was added to the analytical probe and the suspension was stirred at 50°C for 10 minutes. Then it was filtered through paper and it was diluted with 0.05 ml NaOH 2,5M, 0.5 ml acetonitrile and 0.5 ml water (containing 0,1% formic acid)]. The mixtures were analysed using a HPLC.

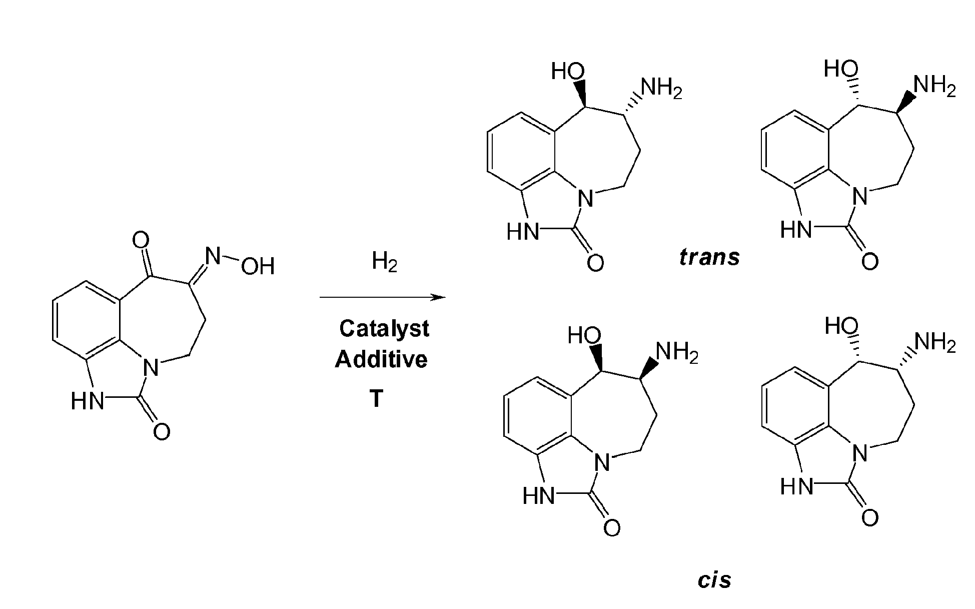

**Table 1: Results with Ru catalysts. The absolute stereochemistry has not been determined. In the table both enantiomers are characterised by positive and negative ee values.**

| Conv. (%) | Aminoalcohol / Hydroxyoxime | Trans / cis | ee (trans;%) | ee (cis;%) | Ligand # | Solvent | Additive | P (bar) | T (°C) |
|---|---|---|---|---|---|---|---|---|---|
| 100 | 4,6/1 | 2,5 | -27 | -12 | 69 | MeOH | KOH | 50 | 50 |
| 72 | 9,1/1 | 2,1 | -6 | 5 | 534 | MeOH | KOH | 50 | 50 |
| 100 | 1,3/1 | 2,9 | -13 | -6 | 534 | MeOH/THF | KOH | 50 | 50 |
| 100 | 1,3/1 | 1,0 | -23 | 1 | 73 | MeOH/THF | KOH | 50 | 50 |
| 84 | 6,9/1 | 1,9 | -12 | -2 | 601 | MeOH | KOH | 50 | 50 |
| 100 | 6,8/1 | 1,6 | -23 | -4 | 601 | MeOH/THF | KOH | 50 | 50 |
| 94 | 11,3/1 | 3,6 | 7 | -9 | 132 | MeOH | KOH | 50 | 50 |
| 100 | 3,2/1 | 3,2 | 3 | 8 | 132 | MeOH/THF | KOH | 50 | 50 |
| 88 | 9,5/1 | 2,8 | -13 | -1 | 607 | MeOH | KOH | 50 | 50 |
| 100 | 3,3/1 | 0,8 | -50 | 82 | 607 | MeOH/THF | KOH | 50 | 50 |
| 100 | 11,2/1 | 1,3 | -9 | 3 | 537 | MeOH | KOH | 50 | 50 |
| 100 | 5,5/1 | 1,1 | -58 | -2 | 688 | MeOH/THF | KOH | 50 | 50 |
| 100 | 14,5/1 | 2,3 | -20 | 10 | 596 | MeOH | KOH | 50 | 50 |
| 100 | 12/1 | 1,7 | -9 | 43 | 1002 | MeOH | KOH | 50 | 50 |
| 89 | 1/0 | 0,7 | 53 | 22 | 732 | MeOH | KOH | 50 | 65 |
| 83 | 609/11 | 0,9 | -15 | 30 | 22 | MeOH | KOH | 50 | 65 |
| 62 | 29,8/1 | 1,0 | -6 | 32 | 89 | MeOH/THF | KOH | 50 | 65 |
| 78 | 1/0 | 0,5 | -51 | -21 | 23 | MeOH | KOH | 40 | 70 |
| 100 | 1/0 | 6,7 | 25 | -4 | 1007 | MeOH | --- | 40 | 70 |

## Claims

1. Process for the enantioselective production of amino alcohols
wherein compounds comprising the general structure (I) 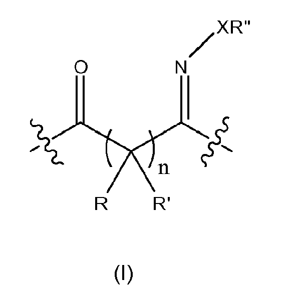 wherein
n is an integer from 0 to 4
X is O or NH
R, R', R" is selected from the group of
H, (C₁-C₁₅)-alkyl, (C₂-C₁₅)-alkenyl, (C₂-C₁₅)-alkynyl, (C₂-C₁₅)-alkoxyalkyl, (C₆-C₁₈)-aryl, (C₇-C₁₉)-aralkyl, (C₃-C₁₈)-heteroaryl, (C₄-C₁₉)-heteroalkyl, (C₁-C₈)-alkyl-(C₆-C₁₈)-aryl, (C₁-C₈)-alkyl-(C₃-C₁₉)-heteroaryl, (C₃-C₈)-cycloalkyl, (C₁-C₈)-alkyl-(C₃-C₈)-cycloalkyl or (C₃-C₈)-cycloalkyl-(C₁-C₈)-alkyl, (C₁-C₈)-Acyloxy, (C₁-C₈)-Acyl,
are reduced in the presence of hydrogen and a metal catalyst comprising a catalytically active metal selected from the group consisting of Ru, Rh, Ir, Pd, Pt and an enantiomerically enriched bidentate ferrocenyl ligand provided that the amino alcohol is not 6-amino-7-hydroxy-4,5,6,7-tetrahydro-imidazo[4,5,1-3K][1]-benzazepin-2[1H]-on or salts thereof.

2. Process according to claim 1;
wherein in general formula (I) at least one of the following features are selected:
n is 0 or 1,
X is O,
R" is H,
R, R' are selected from being H, (C₁-C₁₅)-alkyl, (C₆-C₁₈)-aryl.

3. Process according to claim 1 and/or 2,
wherein the ligand is a compound of general formula (II) 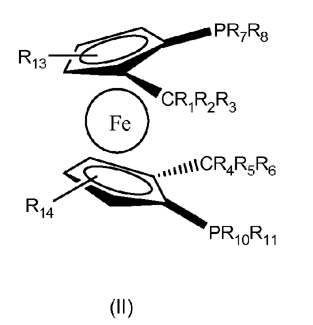 wherein
R₁, R₂, R₃, R₄, R₅, R₆ are selected independently of each other from the group of H, (C₁-C₁₅)-alkyl, (C₂-C₁₅)-alkenyl, (C₂-C₁₅)-alkynyl, (C₂-C₁₅)-alkoxyalkyl, (C₆-C₁₈)-aryl, (C₇-C₁₉)-aralkyl, (C₃-C₁₈)-heteroaryl, (C₄-C₁₉)-heteroalkyl, (C₁-C₈)-alkyl-(C₆-C₁₈)-aryl, (C₁-C₈)-alkyl-(C₃-C₁₉)-heteroaryl, (C₃-C₈)-cycloalkyl, (C₁-C₈)-alkyl-(C₃-C₈)-cycloalkyl or (C₃-C₈)-cycloalkyl-(C₁-C₈)-alkyl, OR, NRR',
R₇, R₈, R₁₀, R₁₁, are selected independently of each other from the group of isopropyl, tert.-butyl, neopentyl, substituted or unsubstituted (C₆-C₁₈)-aryl, substituted or unsubstituted (C₇-C₁₉)-aralkyl, substituted or unsubstituted (C₁-C₈)-alkyl-(C₆-C₁₈)-aryl, substituted or unsubstituted cyclopentyl, substituted or unsubstituted cyclohexyl, substituted or unsubstituted adamantyl.
R₁₃, R₁₄ are selected independently of each other from the group of
H, (C₁-C₁₅)-alkyl, (C₂-C₁₅)-alkoxyalkyl, (C₆-C₁₈)-aryl, (C₇-C₁₉)-aralkyl, (C₃-C₁₈)-heteroaryl, (C₄-C₁₉)-heteroalkyl, (C₁-C₈)-alkyl-(C₆-C₁₈)-aryl, (C₁-C₈)-alkyl-(C₃-C₁₉)-heteroaryl, (C₃-C₈)-cycloalkyl, (C₁-C₈)-alkyl-(C₃-C₈)-cycloalkyl or (C₃-C₈)-cycloalkyl-(C₁-C₈)-alkyl, (C₁-C₈)-Acyloxy, (C₁-C₈)-Acyl,
or display a (C₃-C₅)-alkylene bridge or an annelated aromatic or aliphatic ring system.

4. Process according to any one of the preceding claims,
wherein the metal used is selected from the group of rhodium, ruthenium and iridium.

5. Process according to claim 4,
wherein the metal catalyst is obtained from bis(cyclooctadiene)rhodium(I) tetrafluoroborate, bis(cyclooctadiene)rhodium(I) hexafluorophosphate, bis(cyclooctadiene)rhodium(I) hexafluoroantimonate or bis(norbornadiene)rhodium(I) tetrafluoroborate.

6. Process according to any one of the preceding claims,
wherein the amount of catalyst used is in the range from 0.001 mol% to 10 mol% based on the substrate employed .

7. Process according to any of the preceding claims,
wherein the reduction is performed at a temperature from 20 - 100 °C.

8. Process according to any of the preceding claims,
wherein the reduction is performed at a pressure from 0,1 - 10 MPa.

9. Process according to any of the preceding claims,
wherein the reduction is performed in methanol, THF, dichloromethane, ethanol, isopropanol, dimethylformamide, dimethylacetamide, toluene or mixtures thereof

10. Process according to any of the preceding claims,
wherein an acidic or basic additive is present during the reduction.

11. Process according to any one of the preceding claims,
wherein the hydrogenation reaction is performed as a one-step/one-pot-process.
